# EUROPEAN PATENT APPLICATION

(11) **EP 4 317 311 A1**
(43) Date of publication of application: **07.02.2024**
(21) Application number: 22780347.5
(22) Date of filing: 22.03.2022
(51) Int. Cl.: C08L 45/00, C08L 65/00, A61J 1/05

(54) **CONTAINER**

(30) Priority: 30.03.2021 JP 2021058377
(71) Applicant: Zeon Corporation, Tokyo 100-8246 (JP)
(72) Inventor: SAWAGUCHI, Taichi, Tokyo 100-8246 (JP); HARAUCHI, Yosuke, Tokyo 100-8246 (JP)
(74) Representative: Maiwald GmbH
(86) International application number: PCT/JP2022/013308
(87) International publication number: WO 2022/210132

(57) **Abstract**

Provided is a vessel having reduced non-specific adsorption of protein to an inner wall surface of an accommodating member. The vessel includes an accommodating member obtained through shaping of a shaping material that contains a resin. An inner wall surface of the accommodating member has a zeta-potential at pH 7.0 of less than -40.0 mV and a dispersion term component of surface free energy of less than 24.5 mJ/m².

## Description

### TECHNICAL FIELD

The present disclosure relates to a vessel, and, in particular, relates to a vessel that is used for the purpose of accommodating a protein.

### BACKGROUND

Vessels including accommodating members formed using resin-containing materials are used in a wide range of applications in everyday life. Particularly in the medical field, various vessels such as syringes and infusion bags are used for the purpose of accommodating protein-containing formulations and the like.

Vessels accommodating proteins such as described above have suffered from a problem of non-specific adsorption of protein to an inner wall surface of an accommodating member.

In response to this problem, improvements have been made to vessels with the aim of reducing such non-specific adsorption of protein.

Specifically, Patent Literature (PTL) 1 proposes a technique of setting the zeta-potential of the surface of a vessel that accommodates a sample as the same polarity as a substance that is accommodated as a sample in order to prevent non-specific adsorption of the substance to the surface of the vessel.

### CITATION LIST

### Patent Literature

PTL 1: JP2002-360237A

### SUMMARY

### (Technical Problem)

However, there is room for improvement of the conventional vessel described above in terms of reducing non-specific adsorption of protein to an inner wall surface of an accommodating member.

Accordingly, an object of the present disclosure is to provide a vessel having reduced non-specific adsorption of protein to an inner wall surface of an accommodating member.

### (Solution to Problem)

The inventors conducted diligent investigation with the aim of solving the problem set forth above. The inventors discovered that by using a vessel that includes an accommodating member obtained through shaping of a resin-containing shaping material and in which an inner wall surface of the accommodating member has a zeta-potential at pH 7.0 and a dispersion term component of surface free energy that are less than specific values, it is possible to reduce non-specific adsorption of protein to the inner wall surface of the accommodating member. In this manner, the inventors completed the present disclosure.

Specifically, the present disclosure aims to advantageously solve the problem set forth above, and a presently disclosed vessel comprises an accommodating member obtained through shaping of a shaping material that contains a resin, wherein an inner wall surface of the accommodating member has a zeta-potential at pH 7.0 of less than -40.0 mV and a dispersion term component of surface free energy of less than 24.5 mJ/m². Through a vessel that includes an accommodating member obtained through shaping of a resin-containing shaping material and in which an inner wall surface of the accommodating member has a zeta-potential at pH 7.0 and a dispersion term component of surface free energy that are less than specific values in this manner, it is possible to reduce non-specific adsorption of protein to the inner wall surface of the accommodating member.

Note that the "zeta-potential at pH 7.0" of an inner wall surface of an accommodating member that is referred to in the present disclosure can be measured by a method described in the EXAMPLES section of the present specification.

Also note that the "dispersion term component of surface free energy" of an inner wall surface of an accommodating member that is referred to in the present disclosure can be measured by a method described in the EXAMPLES section of the present specification.

Furthermore, the phrase "accommodating member obtained through shaping of a shaping material that contains a resin" as used in the present disclosure refers to an accommodating member for which at least part of an inner wall thereof is formed of that shaping material.

In the presently disclosed vessel, the resin preferably includes either or both of a hydrogenated cycloolefin ring-opened polymer and a copolymer of a cycloolefin and a chain olefin. When the resin contains either or both of a hydrogenated cycloolefin ring-opened polymer and a copolymer of a cycloolefin and a chain olefin, non-specific adsorption of protein to the inner wall surface of the accommodating member can be further reduced.

In the presently disclosed vessel, the shaping material preferably contains either or both of an antioxidant and a light stabilizer. By using a shaping material that contains either or both of an antioxidant and a light stabilizer, it is possible to further reduce non-specific adsorption of protein to the inner wall surface of the accommodating member.

In the presently disclosed vessel, total content of the antioxidant and the light stabilizer is preferably not less than 0.001 parts by mass and not more than 1.8 parts by mass per 100 parts by mass of the resin. When the ratio of the total amount of the antioxidant and the light stabilizer relative to the resin is within the range set forth above, non-specific adsorption of protein to the inner wall surface of the accommodating member can be further reduced.

Note that the "total content of an antioxidant and a light stabilizer per 100 parts by mass of a resin" that is referred to in the present disclosure can be calculated, for example, using measurement values that are obtained by dissolving an accommodating member of a vessel in an appropriate solvent and subsequently measuring the proportional contents (mass%) of a resin, an antioxidant, and a light stabilizer by high-performance liquid chromatography (HPLC).

### (Advantageous Effect)

According to the present disclosure, it is possible to provide a vessel having reduced non-specific adsorption of protein to an inner wall surface of an accommodating member.

### BRIEF DESCRIPTION OF THE DRAWINGS

In the accompanying drawings:

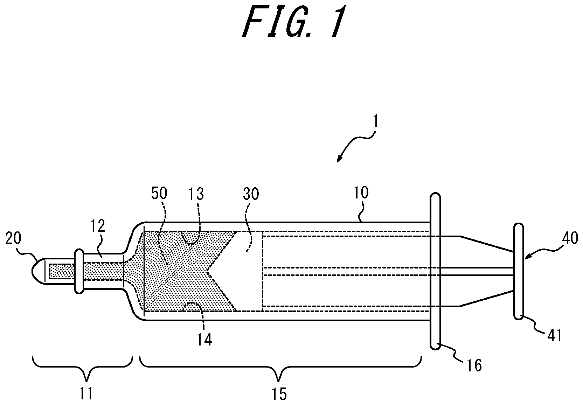

FIG. 1 illustrates schematic configuration of one example of a syringe that is a vessel in accordance with the present disclosure.

### DETAILED DESCRIPTION

The following provides a detailed description of embodiments of the present disclosure.

The presently disclosed vessel is not specifically limited so long as it is a vessel having a purpose of accommodating a protein in an inner part thereof. Specific examples of the presently disclosed vessel include a vial, an infusion bag, a cartridge, an ampoule, a bottle, a tube, a chip, a microwell plate, a pouch, a blister pack, and a syringe.

The following describes the structure of the vessel using a syringe as one example. The syringe includes, for example, a barrel including a nozzle at a tip thereof, a sealing member sealing the nozzle, a gasket slidably housed in the barrel, and a plunger linked to the gasket and performing a movement operation of the gasket in a longitudinal direction of the barrel.

More specifically, one example of the structure of the syringe is illustrated in FIG. 1. A syringe 1 illustrated in FIG. 1 includes a barrel 10, a sealing member 20 (cap in FIG. 1), a gasket 30, and a plunger 40. The barrel 10 includes a nozzle 12 at a tip 11 thereof. The sealing member 20 is fitted to the nozzle 12. The gasket 30 can slide inside the barrel 10 in a longitudinal direction of the barrel 10 and this sliding of the gasket 30 can be performed through the plunger 40 that is linked to the gasket 30. Contained matter 50 is accommodated in the syringe 1. This contained matter 50 is accommodated in a space defined by the sealing member 20, the gasket 30, and a region 14 that is part of an inner wall surface 13 of the barrel 10.

No specific limitations are placed on a protein (corresponding to contained matter 50 in FIG. 1) that is accommodated in the vessel. For example, the protein may be an antibody (chimeric antibody, human antibody, humanized antibody, or domain antibody of any thereof) or an antigen binding fragment of the antibody. Any of these proteins may be present in the form of a solution or may be present in the form of a solid (freeze dried powder, etc.).

More specific examples of the protein include actin, actinin, aggrecan, biglycan, cadherin, clathrin, collagen, decorin, elastin, fibrinogen, fibronectin, heparan, keratin, laminin, mucin, myelin-related glycoprotein, myelin basic protein, myosin, spectrin, tropomyosin, troponin, tubulin, vimentin, vitronectin, ofatumumab (product name: Arzerra^{®} (Arzerra is a registered trademark in Japan, other countries, or both)), cetuximab (product name: Erbitux^{®} (Erbitux is a registered trademark in Japan, other countries, or both)), tocilizumab (product name: Actemra^{®} (Actemra is a registered trademark in Japan, other countries, or both)), bevacizumab (product name: Avastin^{®} (Avastin is a registered trademark in Japan, other countries, or both)), canakinumab (product name: Ilaris^{®} (Ilaris is a registered trademark in Japan, other countries, or both)), golimumab (product name: Simponi^{®} (Simponi is a registered trademark in Japan, other countries, or both)), ustekinumab (product name: Stelara^{®} (Stelara is a registered trademark in Japan, other countries, or both)), eculizumab (product name: Soliris^{®} (Soliris is a registered trademark in Japan, other countries, or both)), omalizumab (product name: Xolair^{®} (Xolair is a registered trademark in Japan, other countries, or both)), trastuzumab (product name: Herceptin^{®} (Herceptin is a registered trademark in Japan, other countries, or both)), pertuzumab (product name: Perjeta^{®} (Perjeta is a registered trademark in Japan, other countries, or both)), adalimumab (product name: Humira^{®} (Humira is a registered trademark in Japan, other countries, or both)), denosumab (product name: Prolia^{®} (Prolia is a registered trademark in Japan, other countries, or both)), mogamulizumab (product name: Poteligeo^{®} (Poteligeo is a registered trademark in Japan, other countries, or both)), rituximab (product name: Rituxan^{®} (Rituxan is a registered trademark in Japan, other countries, or both)), ranibizumab (product name: Lucentis^{®} (Lucentis is a registered trademark in Japan, other countries, or both)), infliximab (product name: Remicade^{®} (Remicade is a registered trademark in Japan, other countries, or both)), and aflibercept (product name: Eylea^{®} (Eylea is a registered trademark in Japan, other countries, or both)).

Note that one type of protein may be used individually, or two or more types of proteins may be used in combination in a freely selected ratio.

### (Vessel)

The presently disclosed vessel includes an accommodating member that can accommodate contained matter. Note that the presently disclosed vessel may include members other than the accommodating member. For example, in a case in which the presently disclosed vessel is a syringe, the syringe can include a sealing member, a gasket, a plunger, and so forth such as illustrated in FIG. 1 in addition to a barrel serving as an accommodating member.

The accommodating member that is included in the presently disclosed vessel is formed through shaping of a shaping material that contains a resin. Features thereof are that an inner wall surface of the accommodating member has a zeta-potential at pH 7.0 of less than -40.0 mV and a dispersion term component of surface free energy of less than 24.5 mJ/m².

In the accommodating member that is included in the presently disclosed vessel, at least part of an inner wall that can come into contact with contained matter such as a protein is formed of a shaping material that contains a resin as previously described. In other words, the shaping material that contains a resin is present at at least part of the inner wall surface of the accommodating member, and this resin-containing shaping material and contained matter such as a protein can be in direct contact without another member (for example, a coating film not formed of the specific shaping material) in-between. In order that the resin-containing shaping material and contained matter such as a protein are in contact in this manner, the entirety of the accommodating member may be formed of the shaping material, or part of the accommodating member may be formed of the shaping material. For example, a wall that forms an internal space of the accommodating member can have a structure (multilayer structure) in which a plurality of layers are stacked and in which at least an innermost layer is formed of the resin-containing shaping material.

From a viewpoint of further reducing non-specific adsorption of protein to the inner wall surface of the accommodating member, it is preferable that for the accommodating member that is included in the presently disclosed vessel, the entirety of an inner wall that can come into contact with contained matter such as a protein is formed of the resin-containing shaping material. Moreover, it is preferable that the presently disclosed vessel does not have a film or layer formed of an organic material and/or an inorganic material on the inner wall surface of the accommodating member, and more preferable that the presently disclosed vessel does not have a film or layer containing silicon (Si) on the inner wall surface of the accommodating member (i.e., the inner wall surface of the accommodating member is not subjected to Si coating or the like). In a case in which the accommodating member has a film or layer such as described above on the inner wall surface thereof, the surface area of a section where the film or layer is present when the total surface area of the inner wall surface of the accommodating member is taken to be 100% is preferably 50% or less, more preferably 25% or less, even more preferably 10% or less, further preferably 5% or less, and particularly preferably 0% (i.e., it is particularly preferable that the presently disclosed vessel does not have a film or layer formed of an organic material and/or an inorganic material on the inner wall surface of the accommodating member).

The presently disclosed vessel has reduced non-specific adsorption of protein to the inner wall surface of the accommodating member as a result of the accommodating member that comes into contact with contained matter such as a protein having an inner wall surface with a zeta-potential at pH 7.0 and a dispersion term component of surface free energy that are less than the previously described values. Although it is not clear why the above-described effect is obtained by using a vessel in which the zeta-potential at pH 7.0 and the dispersion term component of surface free energy of an inner wall surface of an accommodating member satisfy specific conditions in this manner, the reason for this is presumed to be as follows.

First, as a result of the inner wall surface of the accommodating member in the presently disclosed vessel having a zeta-potential at pH 7.0 that is less than -40.0 mV, electrostatic repulsions arise between the inner wall surface and protein molecules having a net charge (effective charge) that is negative at pH 7.0.

In addition, as a result of the inner wall surface of the accommodating member in the presently disclosed vessel having a dispersion term component of surface free energy that is less than 24.5 mJ/m², the content of polar groups at the inner wall surface is small. This inhibits interactions through hydrogen bonds or the like between polar groups in protein molecules and polar groups of the inner wall surface of the accommodating member.

The presence of such electrostatic repulsions and the effect of inhibiting interactions between polar groups are thought to act in conjunction to enable inhibition of non-specific adsorption of protein to the inner wall surface of the accommodating member even when a protein is accommodated in the presently disclosed vessel over a long period.

Accordingly, non-specific adsorption of protein to the inner wall surface of the accommodating member can be reduced when using the presently disclosed vessel.

### <Shaping material>

The shaping material that is used to form the accommodating member included in the presently disclosed vessel contains a resin and may optionally further contain an antioxidant, a light stabilizer, and other components.

### <<Resin>>

The resin that is contained in the shaping material may be a thermoplastic resin that is solid at normal temperature and normal pressure, for example, but is not specifically limited thereto. The thermoplastic resin that is solid at normal temperature and normal pressure may be a hydrogenated cycloolefin ring-opened polymer; copolymer of a cycloolefin and a chain olefin; acrylic resin; silicon resin; fluororesin; polyethylene; polypropylene; ethylene-propylene copolymer; polymethylpentene; polyvinyl chloride; polyvinylidene chloride; polyvinyl acetate; ethylene-vinyl acetate copolymer; polyvinyl alcohol; polyacetal; polyethylene terephthalate; polybutylene terephthalate; polyethylene naphthalate; polystyrene; polyacrylonitrile; styrene-acrylonitrile copolymer; acrylonitrile-butadiene-styrene copolymer (ABS resin); styrene-butadiene block copolymer or hydrogenated product thereof; styrene-isoprene block copolymer or hydrogenated product thereof; polyphenylene ether; modified polyphenylene ether; aliphatic polyamide; aromatic polyamide; polyamide imide; polycarbonate; polyphenylene sulfide; polysulfone; polyethersulfone; polyethernitrile; polyetherketone; polyketone; polyurethane; liquid-crystal polymer; ionomer; or the like.

Of these examples, a hydrogenated cycloolefin ring-opened polymer, a copolymer of a cycloolefin and a chain olefin, polypropylene, polystyrene, or polycarbonate is preferable as the resin from a viewpoint of further reducing non-specific adsorption of protein to the inner wall surface of the accommodating member, a hydrogenated cycloolefin ring-opened polymer, a copolymer of a cycloolefin and a chain olefin, or polystyrene is more preferable as the resin, and a hydrogenated cycloolefin ring-opened polymer or a copolymer of a cycloolefin and a chain olefin is even more preferable as the resin.

Note that in the present specification, "normal temperature" indicates 23°C and "normal pressure" indicates 1 atm (absolute pressure).

The resin that is contained in the shaping material preferably contains either or both of a hydrogenated cycloolefin ring-opened polymer and a copolymer of a cycloolefin and a chain olefin, and more preferably contains at least a hydrogenated cycloolefin ring-opened polymer. By using a resin that contains either or both of a hydrogenated cycloolefin ring-opened polymer and a copolymer of a cycloolefin and a chain olefin, it is possible to further reduce non-specific adsorption of protein to the inner wall surface of the accommodating member.

Note that one type of resin may be used individually, or two or more types of resins may be used in combination in a freely selected ratio.

### [Hydrogenated cycloolefin ring-opened polymer]

The hydrogenated cycloolefin ring-opened polymer is a polymer that is obtained by performing ring-opening polymerization of a cycloolefin as a monomer to obtain a cycloolefin ring-opened polymer, and then further subjecting the obtained cycloolefin ring-opened polymer to a hydrogenation reaction.

### -Cycloolefin ring-opened polymer-

A compound that has a cyclic structure formed of carbon atoms and includes a polymerizable carbon-carbon double bond in the cyclic structure can be used as a cycloolefin serving as a monomer in production of the cycloolefin ring-opened polymer. Specifically, the cycloolefin serving as a monomer may be a norbornene-based monomer (monomer including a norbornene ring) or a monocyclic cycloolefin monomer. Note that in a "norbornene ring" included in a norbornene-based monomer, one or a plurality of carbon atoms may be interposed between carbon-carbon single bonds that form the ring structure, and these interposed carbon atoms may form single bonds with one another, resulting in the formation of a new ring structure in the norbornene ring.

Examples of norbornene-based monomers include:
bicyclic monomers such as bicyclo[2.2.1]hept-2-ene (common name: norbornene) and derivatives thereof (derivatives including a substituent on a ring; same applies below), and 5-ethylidene-bicyclo[2.2.1]hept-2-ene (common name: ethylidene norbornene) and derivatives thereof;
tricyclic monomers such as tricyclo[4.3.0.1^{2,5}]deca-3,7-diene (common name: dicyclopentadiene) and derivatives thereof; and
tetracyclic monomers such as 7,8-benzotricyclo[4.3.0.1^{2,5}]dec-3-ene (common name: methanotetrahydrofluorene; also referred to as tetracyclo[7.4.0.0^{2,7}.1^{10,13}]trideca-2,4,6,11-tetraene) and derivatives thereof, tetracyclo[4.4.0.1^{2,5}.1^{7,10}]dodec-3-ene (common name: tetracyclododecene) and derivatives thereof (for example, 8-methyl-tetracyclo[4.4.0.1^{2,5}.1^{7,10}]dodec-3-ene and 8-ethyl-tetracyclo[4.4.0.1^{2,5}.1^{7,10}]dodec-3-ene), and 8-ethylidenetetracyclo[4.4.0.1^{2,5}.1^{7,10}]-3-dodecene and derivatives thereof.

Examples of possible substituents of the aforementioned derivatives include alkyl groups such as a methyl group and an ethyl group; alkenyl groups such as a vinyl group; alkylidene groups such as an ethylidene group and a propan-2-ylidene group; aryl groups such as a phenyl group; a hydroxy group; an acid anhydride group; a carboxyl group; and alkoxycarbonyl groups such as a methoxycarbonyl group.

Examples of monocyclic cycloolefin monomers include cyclic monoolefins such as cyclobutene, cyclopentene, methylcyclopentene, cyclohexene, methylcyclohexene, cycloheptene, and cyclooctene; and cyclic diolefins such as cyclohexadiene, methylcyclohexadiene, cyclooctadiene, methylcyclooctadiene, and phenylcyclooctadiene.

One of the cycloolefins described above may be used individually, or two or more of the cycloolefins described above may be used in combination. Note that in a case in which two or more cycloolefins are used, the cycloolefin ring-opened polymer may be a block copolymer or may be a random copolymer.

Of these examples, norbornene-based monomers are preferable, tricyclo[4.3.0.1^{2,5}]deca-3,7-diene and derivatives thereof, tetracyclo[4.4.0.1^{2,5}.1^{7,10}]dodec-3-ene and derivatives thereof, and 7,8-benzotricyclo[4.3.0.1^{2,5}]dec-3-ene and derivatives thereof are more preferable, and tricyclo[4.3.0.1^{2,5}]deca-3,7-diene, 8-methyl-tetracyclo[4.4.0.1^{2,5}.1^{7,10}]dodec-3-ene, and 7,8-benzotricyclo[4.3.0.1^{2,5}]dec-3-ene are even more preferable as the cycloolefin.

Although no specific limitations are placed on the amount of a norbornene-based monomer that is used in production of the cycloolefin ring-opened polymer, the amount of the norbornene-based monomer per 100 mass% of the amount of all cycloolefin used in production of the cycloolefin ring-opened polymer is preferably 80 mass% or more, more preferably 90 mass% or more, and even more preferably 100 mass% (i.e., the cycloolefin ring-opened polymer is even more preferably a polymer obtained using only one or more norbornene-based monomers as monomers).

No specific limitations are placed on the method by which the cycloolefin ring-opened polymer is produced. For example, a known method in which a cycloolefin such as described above that is used as a monomer is ring-opening polymerized using a metathesis polymerization catalyst can be adopted. This method may, for example, be a method described in JP2016-155327A.

The weight-average molecular weight (Mw) of the cycloolefin ring-opened polymer obtained as described above is not specifically limited but is preferably 10,000 or more, and more preferably 15,000 or more, and is preferably 100,000 or less, and more preferably 50,000 or less. When the weight-average molecular weight of the cycloolefin ring-opened polymer is 10,000 or more, it is possible to ensure sufficient strength of a vessel obtained using a resin that contains a hydrogenated product of the cycloolefin ring-opened polymer. On the other hand, when the weight-average molecular weight of the cycloolefin ring-opened polymer is 100,000 or less, it is possible to ensure sufficient formability of a resin that contains a hydrogenated product of the cycloolefin ring-opened polymer.

Moreover, the molecular weight distribution (Mw/Mn) of the cycloolefin ring-opened polymer is not specifically limited but is preferably not less than 1 and not more than 5, and more preferably not less than 1 and not more than 4. When the molecular weight distribution of the cycloolefin ring-opened polymer is within any of the ranges set forth above, a vessel having sufficient mechanical strength can be obtained.

Note that the weight-average molecular weight (Mw) and number-average molecular weight (Mn) of a polymer such as a cycloolefin ring-opened polymer referred to in the present disclosure are standard polyisoprene-equivalent values according to gel permeation chromatography (GPC) with cyclohexane as an eluent.

### -Hydrogenation reaction-

The hydrogenated cycloolefin ring-opened polymer can be obtained by subjecting the cycloolefin ring-opened polymer described above to a hydrogenation reaction. No specific limitations are placed on the method by which the cycloolefin ring-opened polymer is hydrogenated. For example, a known method in which hydrogen is supplied into a reaction system in the presence of a hydrogenation catalyst can be adopted. This method may, for example, be a method described in JP2016-155327A.

The percentage hydrogenation in the hydrogenation reaction (proportion of main chain carbon-carbon double bonds that are hydrogenated) is not specifically limited but is preferably 70% or more, more preferably 80% or more, even more preferably 90% or more, and particularly preferably 99% or more from a viewpoint of inhibiting the occurrence of burns and oxidative degradation during production of the accommodating member through shaping of the hydrogenated cycloolefin ring-opened polymer.

Note that the "percentage hydrogenation" in a hydrogenation reaction referred to in the present disclosure can be measured by nuclear magnetic resonance (NMR).

The weight-average molecular weight (Mw) of the hydrogenated cycloolefin ring-opened polymer obtained after the hydrogenation reaction described above is not specifically limited but is preferably 10,000 or more, and more preferably 15,000 or more, and is preferably 100,000 or less, and more preferably 50,000 or less. When the weight-average molecular weight of the hydrogenated cycloolefin ring-opened polymer is 10,000 or more, it is possible to ensure sufficient strength of a vessel obtained using a resin that contains the hydrogenated cycloolefin ring-opened polymer. On the other hand, when the weight-average molecular weight of the hydrogenated cycloolefin ring-opened polymer is 100,000 or less, it is possible to ensure sufficient formability of a resin that contains the hydrogenated cycloolefin ring-opened polymer.

Moreover, the molecular weight distribution (Mw/Mn) of the hydrogenated cycloolefin ring-opened polymer is not specifically limited but is preferably not less than 1 and not more than 5, and more preferably not less than 1 and not more than 4. When the molecular weight distribution of the hydrogenated cycloolefin ring-opened polymer is within any of the ranges set forth above, a vessel having sufficient mechanical strength can be obtained.

### [Copolymer of cycloolefin and chain olefin]

The copolymer of a cycloolefin and a chain olefin (hereinafter, also referred to simply as the "copolymer") is a polymer that is obtained through copolymerization of a cycloolefin as a monomer and a chain olefin as a monomer.

### -Cycloolefin-

Any of the same cycloolefins as previously described in the "Hydrogenated cycloolefin ring-opened polymer" section can be used as the cycloolefin serving as a monomer used in production of the copolymer. One cycloolefin may be used individually, or two or more cycloolefins may be used in combination. Of these cycloolefins, bicyclo[2.2.1]hept-2-ene (common name: norbornene) and derivatives thereof, and tetracyclo[4.4.0.1^{2,5}.1^{7,10}]dodec-3-ene (common name: tetracyclododecene) and derivatives thereof are preferable, and bicyclo[2.2.1]hept-2-ene is more preferable.

### -Chain olefin-

A compound that has a chain structure formed of carbon atoms and includes a polymerizable carbon-carbon double bond in the chain structure can be used as the chain olefin serving as a monomer in production of the copolymer. Note that compounds corresponding to the cycloolefin are not considered to be included among examples of the chain olefin.

The chain olefin may, for example, be an α-olefin such as ethylene, propylene, 1-butene, 1-pentene, or 1-hexene; an aromatic ring vinyl compound such as styrene or α-methylstyrene; or a non-conjugated diene such as 1,4-hexadiene, 4-methyl-1,4-hexadiene, 5-methyl-1,4-hexadiene, or 1,7-octadiene.

One chain olefin may be used individually, or two or more chain olefins may be used in combination. Of these examples, α-olefins are preferable, α-olefins having a carbon number of not less than 1 and not more than 20 are more preferable, and ethylene is even more preferable as the chain olefin.

### -Copolymer-

No specific limitations are placed on the method by which the copolymer is produced. For example, a known method in which the cycloolefin and the chain olefin described above are addition polymerized using a polymerization catalyst can be adopted. This method may, for example, be a method described in JP2016-155327A.

Although no specific limitations are placed on the ratio of amounts of the cycloolefin and the chain olefin used in production of the copolymer, the amount of the cycloolefin per 100 mass% of the total amount of the cycloolefin and the chain olefin used in production of the copolymer is preferably 30 mass% or more, more preferably 50 mass% or more, and even more preferably 70 mass% or more, and is preferably 99 mass% or less, more preferably 97 mass% or less, and even more preferably 95 mass% or less.

Note that the copolymer of the cycloolefin and the chain olefin may be a block copolymer or may be a random copolymer.

The weight-average molecular weight (Mw) of the copolymer of the cycloolefin and the chain olefin is not specifically limited but is preferably 20,000 or more, and more preferably 25,000 or more, and is preferably 100,000 or less. When the weight-average molecular weight of the copolymer is 20,000 or more, it is possible to ensure sufficient strength of a vessel obtained using a resin that contains the copolymer. On the other hand, when the weight-average molecular weight of the copolymer is 100,000 or less, it is possible to ensure sufficient formability of a resin that contains the copolymer.

Moreover, the molecular weight distribution (Mw/Mn) of the copolymer is not specifically limited but is preferably not less than 1 and not more than 5, and more preferably not less than 1 and not more than 4. When the molecular weight distribution of the copolymer is within any of the ranges set forth above, a vessel having sufficient mechanical strength can be obtained.

### <<Antioxidant>>

The antioxidant that can optionally be contained in the shaping material that is used to form the accommodating member included in the presently disclosed vessel may be a phenolic antioxidant, a phosphoric antioxidant, a sulfuric antioxidant, or the like.

The phenolic antioxidant may be pentaerythritol tetrakis[3-(3,5-di-t-butyl-4-hydroxyphenyl)propionate], 3,5-di-t-butyl-4-hydroxytoluene, dibutylhydroxytoluene, 2,2'-methylenebis(6-t-butyl-4-methylphenol), 4,4'-butylidenebis(3-t-butyl-3-methylphenol), 4,4'-thiobis(6-t-butyl-3-methylphenol), α-tocophenol, 2,2,4-trimethyl-6-hydroxy-7-t-butylchromane, tetrakis[methylene-3-(3',5'-di-t-butyl-4'-hydroxyphenyl)propionate]methane, or the like.

The phosphoric antioxidant may be 6-[3-(3-t-butyl-4-hydroxy-5-methylphenyl)propoxy]-2,4,8,10-tetrakis-t-butyldibenzo[d,f][1.3.2]dioxaphosphepine, distearylpentaerythritol diphosphite, bis(2,4-di-tertiary-butylphenyl) pentaerythritol diphosphite, tris(2,4-di-tertiary-butylphenyl) phosphite, tetrakis(2,4-di-tertiary-butylphenyl) 4,4'-biphenyl diphosphite, trinonylphenyl phosphite, or the like.

The sulfuric antioxidant may be distearyl thiodipropionate, dilauryl thiodipropionate, or the like.

Of these examples, phenolic antioxidants are preferable, and pentaerythritol tetrakis[3-(3,5-di-t-butyl-4-hydroxyphenyl)propionate] is more preferable.

Note that one antioxidant may be used individually, or two or more antioxidants may be used in combination in a freely selected ratio.

From a viewpoint of further reducing non-specific adsorption of protein to the inner wall surface of the accommodating member, the content of the antioxidant in the shaping material is preferably 0.001 parts by mass or more, more preferably 0.005 parts by mass or more, and even more preferably 0.01 parts by mass or more per 100 parts by mass of the resin, and is preferably 0.9 parts by mass or less, more preferably 0.5 parts by mass or less, even more preferably 0.3 parts by mass or less, and particularly preferably 0.1 parts by mass or less per 100 parts by mass of the resin.

### <<Light stabilizer>>

The light stabilizer that can optionally be contained in the shaping material used to form the accommodating member included in the presently disclosed vessel may be a hindered amine light stabilizer, a benzoate light stabilizer, or the like.

The hindered amine light stabilizer may be bis(2,2,6,6-tetramethyl-4-piperidyl) sebacate; bis(1,2,2,6,6-pentamethyl-4-piperidyl) sebacate; bis(1,2,2,6,6-pentamethyl-4-piperidyl) [[3,5-bis(1,1-dimethylethyl)-4-hydroxyphenyl]methyl]butylmalonate; a polycondensate of dibutylamine, 2,4,6-trichloro-1,3,5-triazine, N,N'-bis(2,2,6,6-tetramethyl-4-piperidyl)-1,6-hexamethylenediamine, and N-(2,2,6,6-tetramethyl-4-piperidyl)butylamine; or the like.

The benzoate light stabilizer may be 4-benzoyloxy-2,2,6,6-tetramethylpiperidine or the like.

Of these examples, hindered amine light stabilizers are preferable, and a polycondensate of dibutylamine, 2,4,6-trichloro-1,3,5-triazine, N,N'-bis(2,2,6,6-tetramethyl-4-piperidyl)-1,6-hexamethylenediamine, and N-(2,2,6,6-tetramethyl-4-piperidyl)butylamine is more preferable.

Note that one light stabilizer may be used individually, or two or more light stabilizers may be used in combination in a freely selected ratio.

From a viewpoint of further reducing non-specific adsorption of protein to the inner wall surface of the accommodating member, the content of the light stabilizer in the shaping material is preferably 0.01 parts by mass or more, more preferably 0.05 parts by mass or more, and even more preferably 0.1 parts by mass or more per 100 parts by mass of the resin, and is preferably 0.9 parts by mass or less, more preferably 0.5 parts by mass or less, even more preferably 0.3 parts by mass or less, and particularly preferably 0.2 parts by mass or less per 100 parts by mass of the resin.

Moreover, from a viewpoint of further reducing non-specific adsorption of protein to the inner wall surface of the accommodating member, the total content of the antioxidant and the light stabilizer in the shaping material is preferably 0.001 parts by mass or more, more preferably 0.005 parts by mass or more, and even more preferably 0.01 parts by mass or more per 100 parts by mass of the resin, and is preferably 1.8 parts by mass or less, more preferably 1.0 parts by mass or less, even more preferably 0.6 parts by mass or less, and particularly preferably 0.2 parts by mass or less per 100 parts by mass of the resin.

### <<Other components>>

The shaping material used to form the accommodating member included in the presently disclosed vessel can further contain components other than those described above (i.e., other components). For example, the shaping material may contain known additives other than the above-described antioxidants and light stabilizers. Examples of known additives that may be used include ultraviolet absorbers, near-infrared absorbers, plasticizers, antistatic agents, acid scavengers, and the like described in JP2016-155327A, for example. Note that one other component may be used individually, or two or more other components may be used in combination in a freely selected ratio.

### <<Production method of shaping material>>

No specific limitations are placed on the method of mixing adopted when obtaining the shaping material that contains the resin and optionally contains the antioxidant, light stabilizer, and other components described above. For example, mixing can be performed using a known melt-kneading machine such as a single-screw extruder, a twin-screw extruder, a Banbury mixer, a kneader, or a Feeder Ruder.

After mixing, pelletization can be performed in accordance with a standard method by extrusion in a rod form and cutting to an appropriate length using a strand cutter.

### [Zeta-potential]

In the presently disclosed vessel, the inner wall surface of the accommodating member is required to have a zeta-potential at pH 7.0 of less than -40.0 mV. The zeta-potential at pH 7.0 of the inner wall surface of the accommodating member is preferably less than -41.0 mV, more preferably less than -42.0 mV, even more preferably less than -43.0 mV, and particularly preferably less than -44.0 mV. Non-specific adsorption of protein to the inner wall surface of the accommodating member increases in a situation in which the zeta-potential at pH 7.0 of the inner wall surface of the accommodating member is -40.0 mV or more. The lower limit for the zeta-potential at pH 7.0 of the inner wall surface of the accommodating member is not specifically limited and can, for example, be set as more than -60.0 mV, or as more than -55.0 mV.

Note that the zeta-potential at pH 7.0 of the inner wall surface of the accommodating member can be adjusted by, for example, altering the amount (concentration) of the previously described antioxidant and/or light stabilizer. Moreover, the zeta-potential can also be adjusted by, for example, altering the shaping conditions (back pressure, maximum oxygen concentration, etc.) described below in the "Production method of vessel" section.

### [Dispersion term component of surface free energy]

In the presently disclosed vessel, the inner wall surface of the accommodating member is required to have a dispersion term component of surface free energy of less than 24.5 mJ/m². The dispersion term component of surface free energy of the inner wall surface of the accommodating member is preferably less than 23.0 mJ/m², more preferably less than 22.0 mJ/m², even more preferably less than 21.5 mJ/m², further preferably less than 21.0 mJ/m², and particularly preferably less than 20.5 mJ/m². Non-specific adsorption of protein to the inner wall surface of the accommodating member increases in a situation in which the dispersion term component of surface free energy of the inner wall surface of the accommodating member is 24.5 mJ/m² or more. The lower limit for the dispersion term component of surface free energy of the inner wall surface of the accommodating member is not specifically limited and can, for example, be set as more than 10.0 mJ/m², or as more than 15 mJ/m².

Note that the dispersion term component of surface free energy of the inner wall surface of the accommodating member can be adjusted by, for example, altering the amount (concentration) of the previously described antioxidant and/or light stabilizer. Moreover, the dispersion term component of surface free energy can also be adjusted by, for example, altering shaping conditions (back pressure, maximum oxygen concentration, etc.) described below in the "Production method of vessel" section.

### <Production method of vessel>

No specific limitations are placed on the method by which the presently disclosed vessel is produced. For example, the presently disclosed vessel can be produced through a step (shaping step) of shaping the shaping material that contains the resin and optionally contains the antioxidant, light stabilizer, and other components described above to obtain the accommodating member.

### <<Shaping step>>

The method by which the shaping material is shaped is not specifically limited and can be selected as appropriate from known shaping methods depending on the desired shape of the accommodating member. Examples of such known shaping methods include extrusion molding, injection molding, inflation molding, blow molding, extrusion blow molding, injection blow molding, press forming, vacuum forming, powder slush molding, calendering, foam molding, and thermoforming. Of these examples, injection molding is preferably used as the shaping method.

The injection molding is preferably performed using a screw injection molding machine. The screw injection molding machine may, for example, include a cylinder, a screw that is moveable backward and forward in a length direction of the cylinder inside of the cylinder and is rotatable in a direction perpendicular to the length direction of the cylinder, a hopper that is included at a rear section of the cylinder and that stores a feedstock (shaping material) that is to be supplied into the cylinder, and a heater that heats the cylinder. A mold that is in accordance with the desired shape of the accommodating member is connected to a tip of the cylinder at a front section of the cylinder.

The following describes suitable shaping conditions in injection molding using the screw injection molding machine.

### [Back pressure]

In the screw injection molding machine described above, the resin is melted through rotation of the screw inside of the cylinder, which is heated by the heater as necessary, and this molten resin is fed to the front section of the cylinder located further forward than a tip of the screw through the aforementioned rotation. Moreover, when the screw retracts due to the resin that has been fed to the front section of the cylinder located further forward than the tip of the screw, pressure (back pressure) is applied in an opposite direction to the retraction direction of the screw (i.e., is applied in an injection direction). The back pressure in formation of the accommodating member by injection molding is preferably 3 MPa or more, more preferably 5 MPa or more, and even more preferably 7 MPa or more, and is preferably 12 MPa or less, and more preferably 10 MPa or less. When the back pressure in injection molding is 3 MPa or more, formation of polar groups due to oxidative degradation of the vessel is inhibited because the amount of air that becomes mixed in during melting of the shaping material is reduced. Consequently, non-specific adsorption of protein to the inner wall surface of the accommodating member can be further reduced. On the other hand, when the back pressure in injection molding is 12 MPa or less, the shaping material can be shaped in a short time because rotation of the screw of the injection molding machine is not impeded.

### [Maximum oxygen concentration]

The maximum oxygen concentration at a feedstock (shaping material) supply port of the injection molding machine during formation of the accommodating member by injection molding is preferably 7 volume% or less, more preferably 5 volume% or less, and even more preferably 3 volume% or less. When the maximum oxygen concentration is 7 volume% or less, formation of polar groups due to oxidative degradation of the vessel is inhibited. Consequently, non-specific adsorption of protein to the inner wall surface of the accommodating member can be further reduced. The lower limit for the maximum oxygen concentration is not specifically limited and can, for example, be set as 0.05 volume% or more, or as 0.1 volume% or more.

Note that the "maximum oxygen concentration" at the feedstock supply port of the injection molding machine that is referred to in the present disclosure can be measured by a method described in the EXAMPLES section of the present specification.

Moreover, the maximum oxygen concentration can be adjusted by, for example, altering the supply rate at which an inert gas (nitrogen gas, etc.) is fed into the injection molding machine. More specifically, the maximum oxygen concentration can be reduced by increasing the supply rate of the inert gas.

Note that other conditions of injection molding (cylinder temperature, mold temperature, injection rate, injection pressure, screw speed, etc.) can be set as appropriate depending on the type of resin that is used, etc.

### <<Other steps>>

The presently disclosed vessel may optionally be produced through steps other than the shaping step described above (i.e., other steps). Examples of such other steps include a step (pre-drying step) of pre-drying the shaping material in advance of the shaping step and a step (assembly step) of combining the accommodating member obtained by the shaping step and another member so as to assemble the vessel.

### EXAMPLES

The following provides a more specific description of the present disclosure based on examples. However, the present disclosure is not limited to the following examples. In the following description, "%" and "parts" used in expressing quantities are by mass, unless otherwise specified.

In the examples and comparative examples, the following methods were used to measure and evaluate the molecular weight, etc. (weight-average molecular weight, number-average molecular weight, and molecular weight distribution) of a polymer, the percentage hydrogenation when a polymer is hydrogenated, the glass-transition temperature of a polymer, the zeta-potential at pH 7.0, dispersion term component of surface free energy, and amount of fibronectin adsorption for an inner wall surface of an accommodating member, and the maximum oxygen concentration during injection molding.

### <Molecular weight, etc.>

The weight-average molecular weight (Mw) and the number-average molecular weight (Mn) of a polymer were measured as standard polyisoprene-equivalent values by gel permeation chromatography (GPC) with cyclohexane as a solvent. The molecular weight distribution (Mw/Mn) was calculated from the obtained values of Mw and Mn. An HLC-8320GPC (produced by Tosoh Corporation) was used as a measurement apparatus. The standard polyisoprene was standard polyisoprene (monodisperse) produced by Tosoh Corporation, and a total of 10 points were adopted for Mw = 602, 1,390, 3,920, 8,050, 13,800, 22,700, 58,800, 71,300, 109,000, and 280,000. Measurement was performed with a TSKgel^{®} G5000HXL (TSKgel is a registered trademark in Japan, other countries, or both), a TSKgel G4000HXL, and a TSKgel G2000HXL (each produced by Tosoh Corporation) connected in series as a column and under conditions of a flow rate of 1.0 mL/min, a sample injection volume of 100 µL, and a column temperature of 40°C.

### <Percentage hydrogenation>

The percentage hydrogenation in a hydrogenation reaction was calculated through ¹H-NMR measurement with deuterated chloroform as a solvent.

### <Glass-transition temperature (Tg)>

Glass-transition temperature measurement was performed based on JIS K 6911 using a differential scanning calorimeter (produced by SII NanoTechnology Inc.; product name: DSC6220S11).

### <Zeta-potential at pH 7.0>

The zeta-potential of an inner wall surface of a syringe barrel produced in each example or comparative example was determined using a zeta-potential measurement instrument (produced by Anton Paar GmbH; model no.: SurPASS3). First, a 1.0 mM KCl solution was prepared as an electrolyte solution. Next, the syringe barrel was set in a cell for a 1 mL syringe, and then the prepared 1.0 mM KCl solution was injected into the cell. A pH electrode and a conductivity meter were then set up. Titration to pH 7.0 was performed using HCl solution and KOH solution in order to measure the zeta-potential of the syringe barrel at pH 7.0.

### <Dispersion term component of surface free energy>

The surface free energy of an inner wall surface of a syringe barrel produced in each example or comparative example was measured using a contact angle meter (produced by Kyowa Interface Science Co., Ltd.; product name: Drop Master 300). A syringe barrel that had been shaped was cut by diagonal pliers to cut out a solution contacting surface, 2 µL of pure water was dripped thereon, a droplet image was taken 30 seconds thereafter, and the contact angle for pure water was determined by a curve fitting method. In addition, 2 µL of formamide was dripped onto the solution contacting surface, and a droplet image was taken 30 seconds thereafter in order to determine the contact angle for formamide. The dispersion term component γ^{d} of surface free energy was calculated from the obtained contact angles based on the Kaelble-Uy theory.

### <Amount of fibronectin adsorption>

Fibronectin solution (product no. F0895 produced by Sigma-Aldrich; 1 mg/mL solution) was diluted by a factor of 10 using phosphate buffered saline (PBS; pH 7.4; product no. 09-2051-100 produced by Toho Chemical Industry Co., Ltd.) to prepare a 100 µg/mL fibronectin solution. A cap made of isoprene rubber was attached to the tip of a syringe barrel produced in each example or comparative example, and then the barrel was filled with 1.0 mL of the 100 µg/mL fibronectin solution prepared as described above. Next, a plunger having a gasket made of butyl rubber attached thereto was inserted from a rear end of the barrel so as to cause hermetic sealing and obtain a syringe filled with fibronectin solution. The obtained syringe was stored at rest at 4°C for 3 days, and then 1.0 mL of the fibronectin solution was collected. The syringe barrel was washed three times using 1.5 mL of phosphate buffer solution after collection of the fibronectin solution. After washing, the syringe barrel was filled with 1 mL of 3% sodium dodecyl sulfate (SDS) solution and was subjected to 20 minutes of ultrasonication at room temperature so as to collect fibronectin that was adsorbed to the syringe barrel.

The collected fibronectin solution was analyzed by ultra-performance liquid chromatography (UPLC). Fibronectin was adjusted to 5 µg/mL using 3% SDS solution, stepwise dilution was performed by a factor of 2 for each step, and then a calibration curve was prepared from peak areas. The calibration curve was used to calculate the concentration from the peak area obtained through measurement of the collected fibronectin solution, and this concentration was converted to an amount of fibronectin adsorption per 1 m² of the inner wall surface of the syringe barrel. A smaller amount of fibronectin adsorption indicates that there is less non-specific adsorption of protein to an inner wall surface of an accommodating member. The UPLC analysis was performed under the following conditions.

Column: ACQUITY UPLC Protein BEH C4 Column, 300 Angstroms, 1.7 µm, 1 mm × 100 mm (produced by Waters Corporation)
Column temperature: 80°C
Mobile phase A: 0.1 volume% trifluoroacetic acid aqueous solution
Mobile phase B: 0.1 volume% trifluoroacetic acid-containing acetonitrile
Flow rate: 0.09 mL/min

### <Maximum oxygen concentration>

In injection molding of a syringe barrel produced in each example or comparative example, a compact oxygen analyzer (MODEL 3100 produced by Neutronics) was set up at a feedstock (shaping material) supply port of an injection molding machine, the oxygen concentration was measured over time, and a maximum value was taken to be the maximum oxygen concentration (volume%).

### (Example 1)

### <Production of accommodating member (syringe barrel)>

### <<Production of hydrogenated cycloolefin ring-opened polymer (hydrogenated product A)»

In a nitrogen atmosphere, 0.82 parts of 1-dodecene, 0.15 parts of dibutyl ether, and 0.30 parts of triisobutylaluminum were added to 500 parts of dehydrated cyclohexane in a reactor and were mixed therewith at room temperature. Thereafter, the mixture was held at 45°C while 68 parts of tricyclo[4.3.0.1^{2,5}]deca-3,7-diene (common name: dicyclopentadiene; hereinafter, abbreviated as "DCP"), 74 parts of 8-methyl-tetracyclo[4.4.0.1^{2,5}.1^{7,10}]dodec-3-ene (hereinafter, abbreviated as "TCD"), 54 parts of tetracyclo[7.4.0.0^{2,7}.1^{10,13}]trideca-2,4,6,11-tetraene (hereinafter, abbreviated as "MTF"), and 80 parts of tungsten hexachloride (0.7% toluene solution) were continuously added over 2 hours, concurrently to one another, and polymerization was carried out. Next, 1.06 parts of butyl glycidyl ether and 0.52 parts of isopropyl alcohol were added to the polymerization solution to deactivate the polymerization catalyst and stop the polymerization reaction. When the resultant reaction solution containing a ring-opened polymer was analyzed by gas chromatography, the polymerization conversion rate of monomers was 99.5%.

Next, 270 parts of cyclohexane was added to 100 parts of the obtained reaction solution containing the ring-opened polymer, 5 parts of diatomite-supported nickel catalyst (nickel support rate: 58 weight%; pore volume: 0.25 mL/g; specific surface area: 180 m²/g) was further added as a hydrogenation catalyst, the pressure was raised to 5 MPa with hydrogen, heating was performed to a temperature of 200°C under stirring, and then a reaction was carried out for 8 hours to obtain a reaction solution containing a hydrogenated DCP/TCD/MTF ring-opened copolymer. The hydrogenation catalyst was removed by filtration, and then cyclohexane serving as a solvent and other volatile components were removed from the solution at a temperature of 270°C and a pressure of 1 kPa or lower using a cylindrical evaporator (produced by Hitachi, Ltd.). Next, the hydrogenated product was extruded in a strand form from an extruder while in a molten state, was cooled, and was subsequently pelletized to obtain pellets. The hydrogenated cycloolefin ring-opened polymer (hydrogenated product A) that had been pelletized had an Mw of 31,000, a molecular weight distribution (Mw/Mn) of 2.5, a percentage hydrogenation of 99.6%, and a Tg of 135°C.

### <<Production of shaping material (resin pellets) containing hydrogenated product A»

After mixing 100 parts of the hydrogenated product A obtained as described above and 0.012 parts of pentaerythritol tetrakis[3-(3,5-di-t-butyl-4-hydroxyphenyl)propionate] (hereinafter, abbreviated as "antioxidant X") as an antioxidant using a blender, a twin-screw kneader for which hopper purging with nitrogen had been performed was used to knead and extrude the mixture at a cylinder temperature of 255°C to obtain resin pellets.

### <<Shaping>>

The resin pellets obtained as described above were subjected to injection molding under the following conditions while feeding in nitrogen of 99.9% purity as an inert gas at a supply rate of 7 L/min so as to produce a syringe barrel. This injection molding was performed using a mold for a syringe molded product (syringe size: in accordance with 1 mL-Long of ISO standard 11040-6) and an injection molding machine equipped with an inert gas injection device in a hopper (produced by FANUC Corporation; product name: ROBOSHOT aS-50iA). The maximum oxygen concentration during this injection molding was measured.
Cylinder temperature: 310°C
Mold temperature: 105°C
Injection rate: 30 mm/s
Cooling time: 30 s
Injection pressure (holding pressure): 70 MPa
Screw speed: 40 rpm
Back pressure: 8 MPa

The syringe barrel that was obtained in this manner was subjected to measurement and evaluation of the zeta-potential at pH 7.0, the dispersion term component of surface free energy, and the amount of fibronectin adsorption. The results are shown in Table 1.

### (Example 2)

A syringe barrel was produced and various evaluations were performed in the same way as in Example 1 with the exception that in shaping of the syringe barrel, the back pressure during injection molding was changed from 8 MPa to 5 MPa. The results are shown in Table 1.

### (Example 3)

A syringe barrel was produced and various evaluations were performed in the same way as in Example 1 with the exception that in shaping of the syringe barrel, the back pressure during injection molding was changed from 8 MPa to 10 MPa. The results are shown in Table 1.

### (Example 4)

A syringe barrel was produced and various evaluations were performed in the same way as in Example 1 with the exception that in shaping of the syringe barrel, the supply rate of nitrogen was changed from 7 L/min to 5.5 L/min. The results are shown in Table 1.

### (Example 5)

A syringe barrel was produced and various evaluations were performed in the same way as in Example 1 with the exception that in production of the resin pellets containing the hydrogenated product A, the additive amount of the antioxidant X was changed from 0.012 parts to 0.1 parts. The results are shown in Table 1.

### (Example 6)

A syringe barrel was produced and various evaluations were performed in the same way as in Example 1 with the exception that in production of the resin pellets containing the hydrogenated product A, 0.1 parts of a polycondensate of dibutylamine, 2,4,6-trichloro-1,3,5-triazine, N,N'-bis(2,2,6,6-tetramethyl-4-piperidyl)-1,6-hexamethylenediamine, and N-(2,2,6,6-tetramethyl-4-piperidyl)butylamine (hereinafter, abbreviated as "light stabilizer Y") was used instead of 0.012 parts of the antioxidant X. The results are shown in Table 1.

### (Example 7)

A syringe barrel was produced and various evaluations were performed in the same way as in Example 6 with the exception that in production of the resin pellets containing the hydrogenated product A, the additive amount of the light stabilizer Y was changed from 0.1 parts to 0.2 parts. The results are shown in Table 1.

### (Example 8)

### <Production of accommodating member (syringe barrel)>

### <<Production of copolymer of cycloolefin and chain olefin (copolymer B)>>

Under nitrogen flow at normal temperature, norbornene (120 kg) was added into a reactor that had been charged with 258 L of cyclohexane and was stirred for 5 minutes. In addition, triisobutylaluminum was added such that the concentration thereof in the system was 1.0 mL/L. Next, ethylene was circulated at normal pressure while performing stirring in order to convert the system to an ethylene atmosphere. An autoclave internal temperature of 70°C was maintained while raising the internal pressure to a gauge pressure of 6 kg/cm² with ethylene. After 10 minutes of stirring, 0.4 L of a pre-prepared toluene solution containing isopropylidene(cyclopentadienyl)(indenyl)zirconium dichloride and methylalumoxane was added into the system to initiate a copolymerization reaction of ethylene and norbornene. The catalyst concentration relative to the entire system at this point was 0.018 mmol/L of isopropylidene(cyclopentadienyl)(indenyl)zirconium dichloride and 8.0 mmol/L of methylalumoxane. Ethylene was continuously supplied into the system during the copolymerization reaction in order to maintain the temperature at 75°C and the internal pressure at a gauge pressure of 6 kg/cm². After 60 minutes, isopropyl alcohol was added to stop the copolymerization reaction. Depressurization was performed, and then a polymer solution was removed and was brought into contact with an aqueous solution of 5 L of concentrated hydrochloric acid added to 1 m³ of water under vigorous stirring in a ratio of 1:1 to cause catalyst residue to move into the aqueous phase. This contacted liquid mixture was left to settle, the aqueous phase was separated and removed, and washing with water was performed twice to purify and separate a polymerization liquid phase.

The polymerization liquid phase that had been purified and separated was then brought into contact with 3 equivalents of isopropyl alcohol under vigorous stirring to cause precipitation of a copolymer. Thereafter, solid (copolymer) was collected by filtration and was thoroughly washed with isopropyl alcohol. The solid was added into isopropyl alcohol such as to have a concentration of 40 kg/m³, and an extraction operation was subsequently performed under conditions of 3 hours at 70°C in order to extract unreacted monomer. After this extraction, solid was collected by filtration and was dried under circulation of nitrogen at 150°C and 350 mmHg for 10 hours to yield an ethylene-norbornene copolymer (copolymer B). The copolymer B was pelletized in the same way as the hydrogenated product A of Example 1. The pelletized copolymer B had a weight-average molecular weight (Mw) of 95,000, a molecular weight distribution (Mw/Mn) of 2.4, and a Tg of 138°C.

### <<Production of shaping material (resin pellets) containing copolymer B»

After mixing 100 parts of the copolymer B obtained as described above and 0.02 parts of the antioxidant X using a blender, a twin-screw kneader for which hopper purging with nitrogen had been performed was used to knead and extrude the mixture at a cylinder temperature of 250°C to obtain resin pellets.

### <<Shaping>>

A syringe barrel was shaped and various evaluations were performed in the same way as in Example 1 with the exception that the resin pellets containing the copolymer B that were obtained as described above were used and that the conditions of injection molding were changed as indicated below. The results are shown in Table 1.
Cylinder temperature: 300°C
Mold temperature: 105°C
Injection rate: 50 mm/s
Cooling time: 30 s
Injection pressure (holding pressure): 85 MPa
Screw speed: 50 rpm
Back pressure: 9 MPa
Supply rate of inert gas (nitrogen): 7 L/min

### (Example 9)

A syringe barrel was produced and various evaluations were performed in the same way as in Example 8 with the exception that in production of the resin pellets containing the copolymer B, 0.1 parts of the light stabilizer Y was used instead of 0.02 parts of the antioxidant X. The results are shown in Table 1.

### (Example 10)

A syringe barrel was produced and various evaluations were performed in the same way as in Example 1 with the exception that in production of the syringe barrel, polystyrene (Toyo Styrene GPPS G200C produced by Toyo Styrene Co., Ltd.) was used instead of the hydrogenated product A and the conditions of injection molding were changed as indicated below. The results are shown in Table 1.
Cylinder temperature: 280°C
Mold temperature: 85°C
Injection rate: 50 mm/s
Cooling time: 30 s
Injection pressure (holding pressure): 70 MPa
Screw speed: 50 rpm
Back pressure: 8 MPa
Supply rate of inert gas (nitrogen): 7 L/min

### (Example 11)

A syringe barrel was produced and various evaluations were performed in the same way as in Example 10 with the exception that in production of the syringe barrel, 0.1 parts of the antioxidant X was added. The results are shown in Table 1.

### (Comparative Example 1)

A syringe barrel was produced and various evaluations were performed in the same way as in Example 1 with the exception that in production of the resin pellets containing the hydrogenated product A, the additive amount of the antioxidant X was changed from 0.012 parts to 1.0 parts. The results are shown in Table 2.

### (Comparative Example 2)

A syringe barrel was produced and various evaluations were performed in the same way as in Example 1 with the exception that in production of the resin pellets containing the hydrogenated product A, 1.5 parts of the light stabilizer Y was used instead of 0.012 parts of the antioxidant X. The results are shown in Table 2.

### (Comparative Example 3)

A syringe barrel was produced and various evaluations were performed in the same way as in Example 1 with the exception that in shaping of the syringe barrel, the back pressure during injection molding was changed from 8 MPa to 2.5 MPa. The results are shown in Table 2.

### (Comparative Example 4)

A syringe barrel was produced and various evaluations were performed in the same way as in Example 1 with the exception that in shaping of the syringe barrel, the supply rate of nitrogen was changed from 7 L/min to 3 L/min. The results are shown in Table 2.

### (Comparative Example 5)

A syringe barrel was produced and various evaluations were performed in the same way as in Example 8 with the exception that in production of the resin pellets containing the copolymer B, the additive amount of the antioxidant X was changed from 0.02 parts to 1.0 parts. The results are shown in Table 2.

### (Comparative Example 6)

A syringe barrel was produced and various evaluations were performed in the same way as in Example 8 with the exception that in production of the resin pellets containing the copolymer B, 1.5 parts of the light stabilizer Y was used instead of 0.02 parts of the antioxidant X. The results are shown in Table 2.

### (Comparative Example 7)

A syringe barrel was produced and various evaluations were performed in the same way as in Example 8 with the exception that in shaping of the syringe barrel, the back pressure during injection molding was changed from 9 MPa to 2.5 MPa. The results are shown in Table 2.

### (Comparative Example 8)

A syringe barrel was produced and various evaluations were performed in the same way as in Example 8 with the exception that in shaping of the syringe barrel, the supply rate of nitrogen was changed from 7 L/min to 3 L/min. The results are shown in Table 2.

In Tables 1 and 2, shown below:
"Hydrogenated product A" indicates hydrogenated cycloolefin ring-opened polymer (hydrogenated DCP/TCD/MTF ring-opened copolymer);
"Copolymer B" indicates copolymer of cycloolefin and chain olefin (ethylene-norbornene copolymer);
"PS" indicates polystyrene;
"Antioxidant X" indicates pentaerythritol tetrakis[3-(3,5-di-t-butyl-4-hydroxyphenyl)propionate]; and
"Light stabilizer Y" indicates polycondensate of dibutylamine, 2,4,6-trichloro-1,3,5-triazine, N,N'-bis(2,2,6,6-tetramethyl-4-piperidyl)-1,6-hexamethylenediamine, and N-(2,2,6,6-tetramethyl-4-piperidyl)butylamine.

**[Table 1]**

| | | | | | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|---|---|---|
| Vessel | Accommodating member | Shaping material | Resin [parts by mass] | Hydrogenated product A | 100 | 100 | 100 | 100 | 100 | 100 | 100 | 0 | 0 | 0 | 0 |
| | | | | Copolymer B | 0 | 0 | 0 | 0 | 0 | 0 | 0 | 100 | 100 | 0 | 0 |
| | | | | PS | 0 | 0 | 0 | 0 | 0 | 0 | 0 | 0 | 0 | 100 | 100 |
| | | | Antioxidant X [parts by mass] | | 0.012 | 0.012 | 0.012 | 0.012 | 0.1 | 0 | 0 | 0.02 | 0 | 0 | 0.1 |
| | | | Light stabilizer Y [parts by mass] | | 0 | 0 | 0 | 0 | 0 | 0.1 | 0.2 | 0 | 0.1 | 0 | 0 |
| | | Zeta-potential [mV] | | | -46.7 | -45.9 | -47.2 | -42.2 | -44.0 | -40.5 | -40.2 | -41.2 | -40.4 | -47.9 | -46.1 |
| | | Dispersion term component of surface free energy [mJ/m²] | | | 20.0 | 20.2 | 19.9 | 21.2 | 20.8 | 18.5 | 19.2 | 21.5 | 23.1 | 19.0 | 19.5 |
| | Shaping conditions | Cylinder temperature [°C] | | | 310 | 310 | 310 | 310 | 310 | 310 | 310 | 300 | 300 | 280 | 280 |
| | | Mold temperature [°C] | | | 105 | 105 | 105 | 105 | 105 | 105 | 105 | 105 | 105 | 85 | 85 |
| | | Injection rate [mm/s] | | | 30 | 30 | 30 | 30 | 30 | 30 | 30 | 50 | 50 | 50 | 50 |
| | | Injection pressure [MPa] | | | 70 | 70 | 70 | 70 | 70 | 70 | 70 | 85 | 85 | 70 | 70 |
| | | Screw speed [rpm] | | | 40 | 40 | 40 | 40 | 40 | 40 | 40 | 50 | 50 | 50 | 50 |
| | | Back pressure [MPa] | | | 8 | 5 | 10 | 8 | 8 | 8 | 8 | 9 | 9 | 8 | 8 |
| | | Maximum oxygen concentration [volume%] | | | 1.5 | 1.5 | 1.5 | 5 | 1.5 | 1.5 | 1.5 | 1.5 | 1.5 | 1.5 | 1.5 |
| Evaluation | | Amount of fibronectin adsorption [g/m²] | | | 1.6 | 1.7 | 1.6 | 1.9 | 1.7 | 1.9 | 2.0 | 2.0 | 2.3 | 2.3 | 2.4 |

**[Table 2]**

| | | | | | Comparative Example 1 | Comparative Example 2 | Comparative Example 3 | Comparative Example 4 | Comparative Example 5 | Comparative Example 6 | Comparative Example 7 | Comparative Example 8 |
|---|---|---|---|---|---|---|---|---|---|---|---|---|
| Vessel | Accommodating member | Shaping material | Resin [parts by mass] | Hydrogenated product A | 100 | 100 | 100 | 100 | 0 | 0 | 0 | 0 |
| | | | | Copolymer B | 0 | 0 | 0 | 0 | 100 | 100 | 100 | 100 |
| | | | | PS | 0 | 0 | 0 | 0 | 0 | 0 | 0 | 0 |
| | | | Antioxidant X [parts by mass] | | 1.0 | 0 | 0.012 | 0.012 | 1.0 | 0 | 0.02 | 0.02 |
| | | | Light stabilizer Y [parts by mass] | | 0 | 1.5 | 0 | 0 | 0 | 1.5 | 0 | 0 |
| | | Zeta-potential [mV] | | | -39.5 | -27.5 | -36.2 | -38.5 | -38.8 | -25.0 | -34.7 | -36.9 |
| | | Dispersion term component of surface free energy [mJ/m²] | | | 24.8 | 21.6 | 25.2 | 22.5 | 25.0 | 24.0 | 25.8 | 23.4 |
| | Shaping conditions | Cylinder temperature [°C] | | | 310 | 310 | 310 | 310 | 300 | 300 | 300 | 300 |
| | | Mold temperature [°C] | | | 105 | 105 | 105 | 105 | 105 | 105 | 105 | 105 |
| | | Injection rate [mm/s] | | | 30 | 30 | 30 | 30 | 50 | 50 | 50 | 50 |
| | | Injection pressure [MPa] | | | 70 | 70 | 70 | 70 | 85 | 85 | 85 | 85 |
| | | Screw speed [rpm] | | | 40 | 40 | 40 | 40 | 50 | 50 | 50 | 50 |
| | | Back pressure [MPa] | | | 8 | 8 | 2.5 | 8 | 9 | 9 | 2.5 | 9 |
| | | Maximum oxygen concentration [volume%] | | | 1.5 | 1.5 | 1.5 | 7.5 | 1.5 | 1.5 | 1.5 | 7.5 |
| Evaluation | | Amount of fibronectin adsorption [g/m²] | | | 2.8 | 2.6 | 3.0 | 2.5 | 3.2 | 3.0 | 3.4 | 2.8 |

It can be seen from Table 1 that non-specific adsorption of protein to an inner wall surface of an accommodating member is reduced in Examples 1 to 11 in which the used vessel has a zeta-potential at pH 7.0 and a dispersion term component of surface free energy for an inner wall surface of an accommodating member that are less than specific values.

In contrast, it can be seen from Table 2 that non-specific adsorption of protein to an inner wall surface of an accommodating member increases in Comparative Examples 1, 3, 5, and 7 in which the used vessel has a zeta-potential at pH 7.0 and a dispersion term component of surface free energy for an inner wall surface of an accommodating member that are both not less than specific values.

Moreover, it can be seen from Table 2 that non-specific adsorption of protein to an inner wall surface of an accommodating member increases even in Comparative Examples 2, 4, 6, and 8 in which the used vessel has a dispersion term component of surface free energy for an inner wall surface of an accommodating member that is less than a specific value but has a zeta-potential at pH 7.0 for the inner wall surface of the accommodating member that is not less than a specific value.

### INDUSTRIAL APPLICABILITY

According to the present disclosure, it is possible to provide a vessel having reduced non-specific adsorption of protein to an inner wall surface of an accommodating member.

### REFERENCE SIGNS LIST

- 1: syringe
- 10: barrel
- 11: tip
- 12: nozzle
- 13: inner wall surface
- 14: formulation contacting region
- 15: barrel main body
- 16: flange
- 20: sealing member (cap)
- 30: gasket
- 40: plunger
- 41: thumb pad
- 50: contained matter

## Claims

1. A vessel comprising an accommodating member obtained through shaping of a shaping material that contains a resin, wherein an inner wall surface of the accommodating member has a zeta-potential at pH 7.0 of less than -40.0 mV and a dispersion term component of surface free energy of less than 24.5 mJ/m².

2. The vessel according to claim 1, wherein the resin contains either or both of a hydrogenated cycloolefin ring-opened polymer and a copolymer of a cycloolefin and a chain olefin.

3. The vessel according to claim 1 or 2, wherein the shaping material contains either or both of an antioxidant and a light stabilizer.

4. The vessel according to claim 3, wherein total content of the antioxidant and the light stabilizer is not less than 0.001 parts by mass and not more than 1.8 parts by mass per 100 parts by mass of the resin.
